Europäisches Patentamt

European Patent Office

Office européen des brevets

(19)

(11) **EP 1 295 601 A1**

(12) **EUROPÄISCHE PATENTANMELDUNG**
veröffentlicht nach Art. 158 Abs. 3 EPÜ

(43) Veröffentlichungstag:
   **26.03.2003   Patentblatt 2003/13**

(21) Anmeldenummer: 01922165.4

(22) Anmeldetag: **02.04.2001**

(51) Int Cl.$^7$: **A61K 31/616**, A61K 31/194,
   A61K 31/375, A61K 31/7004,
   A61K 35/78, A61P 29/00

(86) Internationale Anmeldenummer:
   **PCT/RU01/00132**

(87) Internationale Veröffentlichungsnummer:
   **WO 01/074367 (11.10.2001 Gazette 2001/41)**

(84) Benannte Vertragsstaaten:
   **AT BE CH CY DE DK ES FI FR GB GR IE IT LI LU
   MC NL PT SE TR**

(30) Priorität: **05.04.2000   RU 2000108267**

(71) Anmelder: **Khazanov, Veniamin Abramovich
   Tomsk, 634034 (RU)**

(72) Erfinder: **Khazanov, Veniamin Abramovich
   Tomsk, 634034 (RU)**

(74) Vertreter: **Jeck, Anton, Dipl.-Ing.
   Patentanwalt,
   Klingengasse 2
   71665 Vaihingen/Enz (DE)**

(54) **ARZNEIFORM AUF DER BASIS EINER NICHTSTEROIDALEN ANTI-INFLAMMATORISCHEN SUBSTANZ**

(57)    Die Erfindung bezieht sich auf den Bereich der Medizin und auf eine pharmazeutische Komposition, die ein steroidfreies entzündungshemmendes Mittel und mindestens eine dessen pharmakologische Wirkung verstärkende und Toxizität und Nebenerscheinungen reduzierende Verbindung enthält, die aus der Gruppe von doppelstämmigen Karbonsäuren oder ihren Salzen, von Aminosäuren oder ihren Salzen, Vitaminen, Polyphenolverbindungen oder polyphenolenthaltenden Produkten vegetarisch und / oder tierischer Herkunft und einem Zielzusatzstoff (Sorbent und / oder Kohlenhydrat) gewählt ist. Die Komposition hat einen stärker ausgeprägten pharmakologischen Effekt und niedrigere Toxizitätskennziffern, auch bei dauernder Einnahme.

**Beschreibung**

Erfindungsgebiet

Die Erfindung betrifft einen Bereich der Medizin, und zwar die Pharmakologie, und hier stereoidfreie entzündungshemmende Mittel (SEM).

Stand der Technik

Die pharmazeutischen Kompositionen der SEM, die in der ärztlichen Praxis gebraucht werden, sind von verschiedener Chemiestruktur z. B. werden zu den SEM die Ableitungen der Salizylsäure (Azetylsalizylsäure, Diflunisal), die Ableitungen der Essigsäure (Indometacin, Sylindac, Tolmetin, Diclofenac), die Ableitungen der Propionsäure (Ibuprofen, Naproxen, Keroprofen, Syrgam), die Ableitungen der Anthranilsäure (Mefenaminsäure, Voltaren), die Ableitungen der Nikotinsäure (Nifluminsäure), Pyrasolone (Butadion, Analgetica, Amidopyrin), Oxicame (Piroxicam) usw. gezählt. Für die pharmazeutischen Kompositionen, die auf der Basis der SEM produziert werden, sind folgende für SEM typische Eigenschaften charakteristisch: eine entzündungshemmende, fiebersenkende Wirkung, auch die Fähigkeit, die Blutgerinnung herabzusetzen. Als negative Wirkung der SEM wird die Ulkuserzeugung der Magenschleimhaut und die toxische Wirkung der Präparate erwähnt, (siehe Charkewitsch D. A. "Pharmakologie", Moskau 1999 S. 461 - 473).

Die bekannte pharmazeutische Komposition auf der Basis der SEM, die oft in der medizinischen Praxis gebraucht wird, ist die Azetylsalizylsäure mit der Askorbinsäure (Aspirin C), wobei die Askorbinsäure die hohe Widerstandsfähigkeit des Organismus und die Senkung der Kapillardurchlässigkeit fördert (siehe Maschkowskij M. D. "Heilmittel", Charkov, Torsing, 1998, B. 1 S. 167)

Zur genannten Komposition steht die Kombination der Azetylsalizylsäure (ASS) mit den Ammoniumsukzinat der Bernsteinsäure in folgender Wechselbeziehung in Massenprozenten am nächsten: ASS 70-80, Ammoniumsukzinat 20-30 (Erfindungspatent Russ. Föd. Nr. 2135185 "Entzündungswidriges Medikament", veröffentlicht in Erfinderbulletin Nr. 24 vom 27.08.1999), aber die Wechselbeziehung der Komponenten und die Anwendung bestimmter Mittel in dieser Komposition erlauben nicht, die pharmakologische Aktivität der SEM zu erhöhen und die Toxizität und Nebenerscheinungen bei der Anwendung der anderen SEM zu reduzieren, die (andere SEM) geringere Heildosen fordern, auch in Verbindung mit anderen Kompositionen, mit denen sich der bedeutendste, therapeutische Effekt ohne Nebenerscheinungen erzielen lässt.

Die Hauptaufgabe, die mit Hilfe der vorliegenden Erfindung gelöst werden kann, ist die Erhöhung der pharmakologischen Aktivität der SEM und deren Kombination mit anderen biologisch aktiven Verbindungen sowie die Beseitigung der Nebenerscheinungen und Reduzierung der Toxizitätseffekte der SEM.

Diese Aufgabe wird dadurch gelöst, dass die pharmazeutische Komposition aus dem SEM und der dessen pharmakologische Wirkung verstärkenden und Nebenerscheinungen und Toxizität reduzierenden Verbindung mindestens eine Verbindung aus folgenden Verbindungen enthält: doppelstämmige Karbonsäuren und ihre Salze, Aminosäuren und ihre Salze, Polyphenolverbindungen oder die polyphenolenthaltenden Produkte vegetarischer und / oder tierischer Herkunft, Stoffe mit Vitaminwirkung und mindestens einen Zielzusatzstoff (Kohlenhydrat und / oder Sorbent).

Als steroidfreie entzündungswidrige Verbindungen kann die Komposition die Azetylsalizylsäure enthalten.

Vorzugsweise enthält die pharmazeutische Komposition Bernsteinsäure als Karbonsäure.

Als Produkt vegetarischer Herkunft, das Polyphenolverbindungen enthält, ist ein Auszug von Bergenium und / oder Auszug von Süssholz (glycyrrhiza) vorteilhaft.

Die pharmazeutische Komposition kann als Zielzusatzstoff Glykose enthalten.

Eine optimale Variante der Erfindungsausführung stellt die folgende Komposition in Massenprozenten dar:

| ASS | 25-80 |
|---|---|
| Ammoniumsukzinat | 5-30 |
| Auszug von Bergenium | 5-20 |
| Askorbinsäure | 5-20 |
| Glykose | Rest |

Die pharmazeutische Komposition kann zusätzlich einen pharmazeutisch passenden Träger enthalten, der für die Oraleinführung geeignet ist. Von Vorteil ist die Ausführung der pharmazeutischen Komposition in Form einer Tablette oder einer Kapsel.

Alle diese Unterscheidungsmerkmale sind für einen Fachmann nicht dem Stand der Technik entnommen. Die technische Ausführung entspricht daher den Kriterien der Erfindung "Neuheit", "Erfindungsniveau". Diese Erfindung kann man in der ärztlichen Praxis als SEM benutzen, weil die SEM einen stärkeren pharmakologischen Effekt und geringere Nebenerscheinungen aufweisen. Diese Ausführung entspricht daher dem Erfindungskriterium "Industrielle Anwendbarkeit".

Die Komposition gemäß der Erfindung besitzt eine stärkere pharmakologische Aktivität, eine niedrigere allgemeine Toxizität- und Nebenerscheinungenmöglichkeit im Vergleich zu analogen Mitteln nach dem Stand der Technik. Das zeigt sich besonders deutlich bei dauernder Einnahme des Präparats. Es lässt einen früher unbekannten positiven Effekt erscheinen.

Als SEM können verschiedenartige Verbindungen aus den oben genannten Gruppen gebraucht werden. Als Agens, das die pharmakologische Aktivität der SEM verstärkt und die Nebenerscheinungen reduziert, können verschiedene pharmakologische geeignete Karbonsäuren in die Komposition eingeführt werden, so z. B. Bernsteinsäure, Fumarsäure, Malleinsäure, Apfelsäure, ihre Kaliumsalze, Natriumsalze, Ammoniumsalze (Ammoniumsukzinat) und andere Salze, Aminosäuren, vorzugsweise Glyzin, Taurin, Glutaminsäure, ihre Salze, Stoffe mit Vitaminwirkung, solche wie Askorbinsäure, Vitamine der B-Gruppe, Retinole und andere, Polyphenolverbindungen vegetarischer und / oder tierischer Herkunft, die rein ausgezogen sind, z. B. Quercetin, Baikalin oder Auszüge von Löwenschweif (leonorus), von Bergenium, von Rastoropscha Pjatnistaja, von Süssholz. Die die Aktivitäten verstärkenden Agenzien werden in effektiver Masse eingeführt, die in Abhängigkeit von der Natur der Agenzien und der Anwendung der Komposition variiert. Die Komposition enthält als Zielzusatzstoff Kohlenhydrate und / oder Sorbente. Sie können die Biozugänglichkeit der Komponenten des kombinierten Präparats (die Veränderung der Auflösfähigkeit der Tablette, die Sorption der Aktivstoffe) beeinflussen, den metabolitischen Effekt hervorrufen (Kohlenhydrate), die Lokalwirkungen der SEM im Magen-Darm-Trakt beschränken. Das alles beweist die Hauptwirkung, nämlich die Verstärkung der entzündungshemmenden Wirkung und die Reduzierung der Toxizität und der Nebenerscheinungen. Als Kohlenhydrate können verschiedene Mono-, Di-, Polyzucker, solche wie Glykose, Fruktose, Sacharose, Laktose, Stachyose und andere, gebraucht werden. Als Sorbent können solche Stoffe wie aktivierte Kohle, Silikagel, Hydroxylapatit, Alyminiumhydroxide, Weizenkleie, Stärke, Mikrokristallzellylose gebraucht werden.

Die oben genannten, bestimmten Stoffe sind für die Darstellung der Erfindung verwendet; sie begrenzen die Erfindung aber nicht.

Ausser den genannten Komponenten kann die Komposition andere in der Pharmazie anerkannte Hilfsmittel einschließen. Diese Mittel lassen die eine oder andere Form der Einführung der Komposition wählen. Es können Lösungs-, Glatt-, Pudermittel, Geschmacksstoffe u.a. sein.

Die Kompositionen können verschiedene Formen haben. Vorzugsweise sind für die Oraleinführung Lösungen, Sirupe, Pulver, Tabletten, Kapseln geeignet.

Beschreibung der Realisierungsvarianten der Erfindung

Die beschriebenen Merkmale der Komposition gemäß der Erfindung wurden durch die Versuche mit Tieren und durch die medizinische Praxis bewiesen. Die Versuche sind in folgenden Beispielen dargelegt:

**Beispiel 1:** Die Verstärkung der entzündungswidrigen Wirkung der SEM

Es wurde die Wirkung folgender möglicher Komponenten der Komposition auf die entzündungshemmende Aktivität getestet:

A) Die Wirkung der Auszüge mit hohem Gehalt von Polyphenolverbindungen: des Auszugs von Baikal-Helmkraut (ABH) und des Auszugs von Bergenium (AB), der Polyphenolverbindungen mit Flavonoidstruktur-Silibinin (SB), Quercetin (QU).

B) Die Wirkung der Karbonsäuren - der Apfelsäure (APF), der Fumarsäure (FUM), der Bernsteinsäure (BS) und ihres Ammoniumsalzes (BS-$NH_4$).

C) Die Wirkung der Aminosäuren - der Glutaminsäure (GLU), des Glyzins (GLY), des Zysteins (ZYS), des Taurins (TAU).

D) Die Wirkung der Stoffe mit Vitaminwirkung - Vitamine C, $B_6$, E, Liponsäure (LS).

Als SEM wurden die Verbindungen aus allen Hauptgruppen folgender Präparate benutzt: Azetylsalizylsäure (ASS), Indometacin (IM), Naproxen (NP), Voltaren (VL), Nifluminsäure, (NS), Butadion (BN), Piroxicam (PC). Die einmalige Dosis der SEM variiert von 10mg (für Piroxicam) bis 500mg (für Aspirin) und hängt von der Art des Präparats, von der Schwere der Krankheit, vom Alter und vom Gewicht des Kranken ab.

Die Versuche wurden mit weißem, nicht reinrassigen Mäusen (Männchen mit einem Gewicht von 22 - 24g) durchgeführt. Die zu untersuchenden Präparate wurden den Tieren durch eine Sonde in den Magen einmal pro Tag innerhalb von 5 Tagen in Form einer Suspension aus 1 Prozent Stärkeschleim oder einer Wasserlösung eingeführt. Um die Dosis des zu benutzenden Präparats zu wählen, wurden die aus der Literatur bekannten Werte verwendet. Diese Werte sind Ergebnisse der Tierversuche oder Ergebnisse der medizinischen Untersuchung der Menschen.

Dabei wird die vom Ministerium für Gesundheitswesen empfohlene Methodik der Umrechnung eingesetzt. (Die Aufführungsregeln der vorklinischen Untersuchungen in Pharmakokinetik der Heilmittel, Moskau, 1998, S. 12-13).

ASS wurde für die Mäuse optimal dosiert benutzt, nämlich 200mg/kg (s. Maschkowskij M.D. "Heilmittel", Charkov, Torsing 1998, Band 1, S. 165-167), die anderen SEM wurden folgenderweise dosiert (Die Umrechnung der therapeutischen Dosis für Menschen wurde berücksichtigt): IM (10) (s. Maschkowskij M.D. "Heilmittel", Charkov, Torsing 1998, Band 1, S. 173), NP (100) (s. Maschkowskij M.D. "Heilmittel", Charkov, Torsing, 1998, Band 1, S 174), VL (10) (s. Maschkowskij M.D. "Heilmittel", Charkov, Torsing, 1998, Band 1 S. 172), NS (100) (s. Maschkowskij M.D. "Heilmittel", Charkov, Torsing, 1998, Band 1 S. 176), BN (60) (s. Maschkowskij M.D. "Heilmittel", Charkov, Torsing, 1998, Band 1, S 169-171), PC (8) (s. Maschkowskij M.D. "Heilmittel", Charkov, Torsing, 1998, Band 1 S. 174).

Die die Aktivität der SEM verstärkenden Verbindungen werden in effektiven Mengen eingeführt, die in Abhängigkeit von ihrer Natur und dem Anwendungsziel der Komposition variieren. So werden die Karbonsäuren in einer Menge von 5mg bis 500mg für die einmalige Dosis eingeführt. Die Menge der Aminosäure kann von 0,1 mg (für Taurin) bis 500mg (für Glutaminsäure) variieren. Die Vitamine sind in der Komposition in biologisch aktiven Dosen vorhanden, die von 2mg (für Riboflavin) bis 500mg (für Askorbinsäure) enthalten sein können. Die Polyphenolverbindungen sind in der Regel in einer Menge von 2mg (für Rutin) bis 300mg (für den Löwenschweifauszug) in der Komposition enthalten.

Bei den bestimmten Versuchen, die in den Beispielen dargestellt sind, waren die Dosen der Karbonsäuren und ihrer Salze folgende: BS-Na (100), BS-NH$_4$ (100), APF (100) (s. "Therapeutische Wirkung der Bernsteinsäure", unter der Redaktion von M.N. Kondraschowa, Puschtschino, 1976, S. 49-55, S. 77-79), FUM (100) (s. Ueda H. et al., "Reduction of cisplatin toxicity and lethality by sodium malate in mice", Biol Pharm Bull., 1998 Lan, 21:1, 34-43).

Die Dosen der Aminosäure waren: GLU (100) (s. Maschkowskij M.D., "Heilmittel", Charkov, Torsing. 1998, Band 2, S. 128-129), GLY (100) (s. Maschkowskij M.D., "Heilmittel", Charkov, Torsing. 1998, Band 2, S. 131), ZYS (50), (s. Maschkowskij M.D., "Heilmittel", Charkov, Torsing. 1998, Band 2, S. 131), TAU (50) (s. Maschkowskij M.D., "Heilmittel", Charkov, Torsing. 1998, Band 2, S. 132).

Die Dosen der Polyphenolverbindungen oder der polyphenolenthaltenden Auszüge waren: ABH = 150mg/kg (s. Sajfutdinow R.R. "Einfluss des Baikal-Helmkrauts auf energetische Metabolie des Gehirns der Ratten bei Hypoxie", Autoreferat der Dissertation des Kandidaten der Medizinwissenschaft, Tomsk, 1997, 235), AB = 100mg/kg (s. Chasanow V.A., Smirnowa N.B., Iluschenko S.V., "Mitochondrialeffekte im Mechanismus der zerebroschützenden Wirkung des Auszugs von Bergenium bei Hypoxie", Bulletin der Experimentbiologie und Medizin, 2001, Beilage 1, S. 34-38), Quercetin = 50mg/kg (s. Maschkowskij M.D., "Heilmittel", Charkov, Torsing, 1998, Band 2, S. 91), Silibinin = 50mg/kg (s. Maschkowskij M.D., "Heilmittel", Charkov, Torsing, 1998, Band 1, S. 514-515).

Die Dosen der Vitamine waren Vit. C (50) (s. Maschkowskij M.D., "Heilmittel", Charkov, Torsing, 1998, Band 2, S. 88-89), Vit. B$_6$ (10) (s. Maschkowskij M.D., "Heilmittel", Charkov, Torsing, 1998, Band 2, S. 80-81), Vit. E (20) (s. Maschkowskij M.D., "Heilmittel", Charkov, Torsing, 1998, Band 2, S. 95-96), Liponsäure (20) (s. Maschkowskij M.D., "Heilmittel", Charkov, Torsing, 1998, Band 2, S. 104).

Eine Stunde nach der letzten Einführung der Präparate wurden die Mäuse mit 0,05ml 1-prozentiger Karrageninlösung in einer isotonischen Lösung NaCl unter die Aponeurose der rechten Hinterpfote geimpft. Nach 3,5 Stunden, auf dem Entzündungshöhepunkt, wurden die Mäuse durch Verschiebung der Halswirbel getötet. Es wurden die Massen der gesunden und der angeschwollenen Pfote verglichen und der Grad der Ödemunterdrückung in der Testgruppe im Vergleich zur Kontrollgruppe nach folgender Formel bestimmt:

$$\frac{\text{Massenzunahme der Pfote in der Kontrollgruppe minus Massenzunahme der Pfote in der Testgruppe}}{\text{Massenzunahme in der Kontrollgruppe}} \times 100.$$

Die Ergebnisse wurden statistisch bearbeitet. Es wurde das nichtparametrische Kriterium von Vilkokson benutzt (s. Belenjkij M.L. "Die Elemente der Zahleinschätzung des pharmakologischen Effekts", 2. Ausgabe, bearb. und erweit. von L. Medgis, 1963, S. 152).

In der Tabelle 1 sind die Ergebnisse der Wirkung der unterschiedlichen Präparate aufgeführt. Es sei betont, dass alle SEM die Entwicklung der Entzündungsreaktion bestimmt begrenzen. Die übrigen Kompositionskomponenten haben keine phlogolitische Reaktion gezeigt.

Tabelle 1

| Der Einfluss der Untersuchungspräparate auf die Entwicklung des 20 Entzündungsödems in der Mauspfote nach einer Karrageninimpfung (n = 10) | | |
|---|---|---|
| Testgruppe | Massenzunahme der Pfote, mg | Ödemunterdrückung in Prozenten vgl. mit Kontrollgruppe |
| Kontrollgruppe | 60.1 ± 7,4 | - |
| ASS (200) | 41,3 ± 5,2 | -31,3* |
| Im (10) | 19,0 ± 6,3 | -68,3* |
| NP (100) | 28,9 ± 5,4 | -51,9* |
| VL (10) | 24,3 ± 5,8 | -59,5* |
| NS (100) | 31,1 ± 6,0 | -48,3* |
| BN (60) | 32,4 ± 5,2 | -46,1* |
| PC (8) | 28,8 ± 4,9 | -52,0* |
| BS (100) | 59,6 ± 5,8 | -0,8 |
| BS-NH$_4$ (100) | 63,5 ± 6,1 | +5,7 |
| BS-Na (100) | 61,7 ± 4,9 | +2,7 |
| APF (100) | 58,4 ± 6,3 | -2,8 |
| FUM (100) | 59,3 ± 5,5 | -1,3 |
| GLU (100) | 62,3 ± 5,0 | +3,7 |
| GLY (100) | 63,0 ± 5,5 | +4,8 |
| ZYS (50) | 61,5 ± 6,4 | +2,3 |
| TAU (50) | 60,0 ± 5,6 | -0,2 |
| ABH (150) | 59,4 ± 3,0 | -1,2 |
| AB (100) | 60,5 ± 6,1 | -0,7 |
| QU (50) | 58,7 ± 3,6 | -2,3 |
| SB (50) | 62,3 ± 6,6 | +3,7 |
| Vit. C (50) | 59,4 | -1,2 |
| Vit. B$_6$ (10) | 60,5 | +0,7 |
| Vit. E (20) | 58,7 | -2,3 |
| Liponsäure | 62,3 | +3,7 |
| Anmerkung: hier und weiter (*)- Unterschied zur Kontrollkennziffer in seiner Gruppe | | |

Tabelle 2

| Der Einfluss der Aminosäure auf die entzündungshemmende Aktivität des SEM bei der Entwicklung des Pfotenödems der Mäuse nach einer Karrageninimpfung (n=10) | | | | | |
|---|---|---|---|---|---|
| Präparat | Kontrolle | GLU(100) | GLY(100) | ZYS (50) | TAU (50) |
| Kontrolle | 60,1 | 59,4 | 60,5 | 58,7 | 62,3 |
| ASS (200) | 41,3* | 37,3*^ | 36,0*^ | 34,9*^ | 35,3*^ |
| IM (10) | 19,0* | 16,0*^ | 15,2*^ | 15,4*^ | 15,8*^ |
| NP (100) | 28,9* | 24,4*^ | 25,2*^ | 23,1*^ | 24,0*^ |

Tabelle 2 (fortgesetzt)

| Der Einfluss der Aminosäure auf die entzündungshemmende Aktivität des SEM bei der Entwicklung des Pfotenödems der Mäuse nach einer Karrageninimpfung (n=10) | | | | | |
|---|---|---|---|---|---|
| Präparat | Kontrolle | GLU(100) | GLY(100) | ZYS (50) | TAU (50) |
| VL (10) | 24,3* | 19,5*^ | 20,7*^ | 19,0*^ | 19,3*^ |
| NS (100) | 31,1* | 25,7*^ | 25,2*^ | 24,0*^ | 25,9*^ |
| BN (60) | 32,4* | 27,6*^ | 26,1*^ | 26,6*^ | 27,0*^ |
| PC (8) | 28,8* | 24,0*^ | 23,2*^ | 23,3*^ | 24,5*^ |
| Anmerkung: hier und weiter (^) - Unterschied zu den entsprechenden Kennziffern in der Kontrollsparte. | | | | | |

Tabelle 3

| Der Einfluss der Polyphenolverbindungen auf die entzündungshemmende Aktivität des SEM bei der Entwicklung des Pfotenödems der Mäuse nach einer Karrageninimpfung. | | | | | |
|---|---|---|---|---|---|
| Präparat | Kontrolle | ABH(150) | AB(100) | QU(50) | SB(50) |
| Kontrolle | 60,1 | 59,4 | 60,5 | 58,7 | 62,3 |
| ASS (200) | 41,3* | 37,3*^ | 36,0*^ | 34,9*^ | 35,3*^ |
| IM (10) | 19,0* | 16,0*^ | 15,2*^ | 15,4*^ | 15,8*^ |
| NP (100) | 28,9* | 24,4*^ | 25,2*^ | 23,1*^ | 24,0*^ |
| VL (10) | 24,3* | 19,5*^ | 20,7*^ | 19,0*^ | 19,3*^ |
| NS (100) | 31,1* | 25,7*^ | 25,2*^ | 24,0*^ | 25,9*^ |
| BN (60) | 32,4* | 27,6*^ | 26,1*^ | 26,6*^ | 27,0*^ |
| Pc (8) | 28,8* | 24,0*^ | 23,2*^ | 23,3*^ | 24,5*^ |

Tabelle 4

| Der Einfluss der Karbonsäuren auf die entzündungshemmende Aktivität der SEM bei der Entwicklung des Pfotenödems der Mäuse nach einer Karrageninimpfung (n = 10). | | | | | |
|---|---|---|---|---|---|
| Präparat | Kontrolle | BS-NH$_4$(100) | BS-Na (100) | APF (100) | FUM (100) |
| Kontrolle | 60,1 | 63,5 | 61,7 | 58,7 | 59,3 |
| ASS (200) | 41,3* | 37,3*^ | 36,0*^ | 34,9*^ | 35,3*^ |
| IM (10) | 19,0* | 15,7*^ | 16,4*^ | 16,1*^ | 15,0*^ |
| NP (100) | 28,9* | 24.2*^ | 24,9*^ | 24,0*^ | 25,1*^ |
| VL (10) | 24,3* | 19,9*^ | 20,0*^ | 19,2*^ | 19,5*^ |
| NS (100) | 31,1* | 26,8*^ | 26,0*^ | 25,5*^ | 26,7*^ |
| BN (60) | 32,4* | 28,0*^ | 27,3*^ | 26,2*^ | 26,6*^ |
| PC (8) | 28,8* | 24,4*^ | 24,3*^ | 23,9*^ | 24,0*^ |

Tabelle 5

| Der Einfluss der Vitaminpräparate auf die entzündungshemmende Aktivität der SEM bei der Entwicklung des Pfotenödems der Mäuse nach einer Karrageninimpfung (n = 10). | | | | | |
|---|---|---|---|---|---|
| Präparat | Kontrolle | Vit. C (50) | Vit. B6(10) | Vit. E (20) | LS (20) |
| Kontrolle | 60,1 | 59,4 | 60,5 | 58,7 | 62,3 |

Tabelle 5   (fortgesetzt)

| Der Einfluss der Vitaminpräparate auf die entzündungshemmende Aktivität der SEM bei der Entwicklung des Pfotenödems der Mäuse nach einer Karrageninimpfung (n = 10). | | | | | |
|---|---|---|---|---|---|
| Präparat | Kontrolle | Vit. C (50) | Vit. B6(10) | Vit. E (20) | LS (20) |
| ASS (200) | 41,3* | 38,0*^ | 37,2*^ | 35,5*^ | 36,2*^ |
| IM (10) | 19,0* | 16,6*^ | 16,0*^ | 15,0*^ | 15,3*^ |
| NP (100) | 28,9* | 25,2*^ | 25,6*^ | 24,4*^ | 25,1*^ |
| VL (10) | 24,3* | 20,9*^ | 20,2*^ | 19,6*^ | 19,2*^ |
| NS (100) | 31,1* | 26,6*^ | 25,9*^ | 24,8*^ | 26,0*^ |
| BN (60) | 32,4* | 28,1*^ | 27,3*^ | 26,8*^ | 27,2*^ |
| PC (8) | 28,8* | 24,4*^ | 23,8*^ | 24,0*^ | 23,3*^ |

Tabelle 6

| Der Einfluss der Polyphenolverbindungen oder der polyphenolenthaltenden Pflanzenauszüge auf die entzündungshemmende Aktivität der Kombination der SEM mit Karbonsäuren, Aminosäuren und Vitaminen bei der Entwicklung des Pfotenödems der Mäuse nach einer Karrageninimpfung (n = 10). | | | | | |
|---|---|---|---|---|---|
| Präparat | Kontrolle | ABH(150) | AB(100) | QU(50) | SB(50) |
| Kontrolle | 60,1 | 59,4 | 60,5 | 58,7 | 62,3 |
| ASS (200)+ BS-NH$_4$ (100) | 37,3* | 33,0*^ | 32,8*^ | 30,0*^ | 31,4*^ |
| IM (10)+ FUM (100) | 15,0* | 12,1*^ | 11,6*^ | 12,7*^ | 11,2*^ |
| NP (100)+ Vit.C (50) | 25,2* | 20,7*^ | 21,0*^ | 20,5*^ | 22,0*^ |
| VL(10)+ Vit.B$_6$ (10) | 20,2* | 16,3*^ | 15,8*^ | 16,2*^ | 15,0*^ |
| BN (60)+ GLU (100) | 28,4* | 22,0*^ | 21,9*^ | 22,8*^ | 23,6*^ |
| PC(8)+ GLY (100) | 24,3* | 19,3*^ | 18,2*^ | 17,9*^ | 18,0*^ |

Wie die Tabellen 2-5 beweisen, erhöhen die Polyphenolverbindungen und die polyphenolenthaltenden Pflanzenauszüge, Karbonsäuren, Aminosäuren und Vitamine die entzündungshemmende Aktivität des SEM. In der Tabelle 6 sind Werte für die Verstärkung der entzündungshemmenden Aktivitäten der Komposition, die SEM, das Vitamin oder die Karbonsäure oder die Aminosäure dargestellt.

**Beispiel 2:** Die Einschätzung des Einflusses der Kohlenhydrate und der Stoffe mit Sorbtionsfähigkeiten auf die Toxizität von ASS.

Die Versuche wurden mit weißen, nicht reinrassigen Mäusemännchen mit einem Gewicht von 22-26g durchgeführt. Die hohe Toxizität von ASS wurde nach der Grösse der Dosis LD 50 (diese verursacht den Tod von 50 Prozent der Tiere) der Komposition mit ASS und Kohlenhydrat oder Sorbent in Wechselbeziehung 4:1 im Vergleich zu LD 50 von ASS gewählt. Die Präparate wurden einmalig in den Magen in Form einer Wassersuspension eingeführt.
Die Dosen von 350-3000mg/kg gelten für die Gruppen mit je 6 Mäusen. Es wurden aktivierte Kohle, Kaolin, Alyminiumhydroxide, Magnesiumtrisilikat, Hydroxylapatit, Mikrokristallzellylose (MKZ) benutzt. Als Kohlenhydrat wurden Monozucker-Glykose, Fruktose, Dizucker-Sacharose, Laktose, Polysacharid-Kartoffelstärke benutzt. Die Ergebnisse des Experiments ergaben sich nach der Methodik von Lichfield-Vilkokson (s. Belenikij M.L. "Element der Zahleinschätzung des pharmakologischen Effekts", 2. Ausgabe, bearb. und erweit. von L. Medgis, 1963, S. 152). Das LD 50 der Komposition ist in ASS gerechnet (Tabellen 7, 8).

Tabelle 7

| Der Einfluss der Stoffe mit Sorptionsfähigkeiten auf die hohe Toxizität von ASS (n = 6). | | |
|---|---|---|
| Präparat | LD 50 mg/kg in ASS | Toxizitätsreduzierung in |
| ASS | 2340 | - |
| ASS + aktive Kohle | 2612 | 1,12 |
| ASS + Kaolin | 2756 | 1,18 |
| ASS + Alyminiumhydroxide | 2695 | 1,15 |
| ASS + Magnesiumtrisilikat | 2574 | 1,10 |
| ASS + Hydroxylapatit | 2638 | 1,13 |
| ASS+MKZ | 2682 | 1,15 |

Tabelle 8

| Der Einfluss der Kohlenhydrate auf die hohe Toxizität von ASS. | | |
|---|---|---|
| Präparat | LD 50 mg/kg in ASS | Toxizitätsreduzierung in |
| ASS | 2340 | - |
| ASS + Glykose | 2542 | 1,09 |
| ASS + Fruktose | 2620 | 1,12 |
| ASS + Sacharose | 2530 | 1,15 |
| ASS + Laktose | 2632 | 1,08 |
| ASS + Kartoffelstärke | 2816 | 1,20 |

Wie die dargelegten Werte zeigen, reduzieren die Stoffe mit Sorptionsfähigkeiten und Kohlenhydrate in gemeinsamer Anwendung mit SEM ihre (ASS) Toxizität.

**Beispiel 3:** Die Einflusseinschätzung der Kohlenhydrate und Stoffe mit Sorptionsfähigkeiten in Dosen von 100mg/kg auf die pharmakologische Aktivität der SEM in der Kombination mit der die Wirkung der SEM verstärkenden Verbindung

Diese Verbindung ist aus folgender Gruppe gewählt: doppelstämmige Karbonsäuren, ihre Salze, Aminosäuren, Polyphenolverbindungen, die polyphenolenthaltenden Produkte vegetarischer oder tierischer Herkunft, Vitamine. Die Dosen der benutzten Verbindungen sind in der Tabelle aufgeführt und in mg/kg ausgedrückt. Die Versuche wurden mit weißen, nicht reinrassigen Mäusemännchen mit einem Gewicht von 22-26g durchgeführt.

Tabelle 9

| Der Einfluss der Sorbente und Kohlenhydrate auf die entzündungshemmende Aktivität der Kombination der SEM mit Karbonsäuren, Aminosäuren, Vitaminen, Polyphenolverbindungen bei der Entwicklung des Pfotenödems der Mäuse nach einer Karrageninimpfung (n = 10) | | | | | | | |
|---|---|---|---|---|---|---|---|
| Präparat | Kontr. | AK | MTS | MKZ | Glykose | LAK | FRU |
| Kontrolle | 60,1 | 62,2 | 58,3 | 61,7 | 63,0 | 58,8 | 61,1 |
| ASS (200) + BS/NH$_4$ (100) | 37,3* | 35,5*^ | 36,0*^ | 34,7*^ | 35,1*^ | 36,2*^ | 35,4*^ |
| IM(10)+ FUM(100) | 15,0* | 13,6*^ | 12,9*^ | 12,6*^ | 12,8*^ | 14,1*^ | 13,5*^ |

Tabelle 9 (fortgesetzt)

| Der Einfluss der Sorbente und Kohlenhydrate auf die entzündungshemmende Aktivität der Kombination der SEM mit Karbonsäuren, Aminosäuren, Vitaminen, Polyphenolverbindungen bei der Entwicklung des Pfotenödems der Mäuse nach einer Karrageninimpfung (n = 10) | | | | | | | |
|---|---|---|---|---|---|---|---|
| Präparat | Kontr. | AK | MTS | MKZ | Glykose | LAK | FRU |
| NP(100)+ Vit.C (50) | 25,2* | 23,0*^ | 22,3*^ | 24,0*^ | 22,9*^ | 22,2*^ | 22,0*^ |
| VL(10)+ Vit.B$_6$ (10) | 20,2* | 18,4*^ | 17,8*^ | 16,9*^ | 17,7*^ | 18,5*^ | 19,0*^ |
| BN(60) + GLU(100) | 28,4* | 26,3** | 27,0*^ | 25,9*^ | 26,7*^ | 25,2*^ | 26,4*^ |
| PC(8)+ GLY(100) | 24,3* | 20,5*^ | 21,2*^ | 22,0*^ | 22,5*^ | 21,6*^ | 22,9*^ |
| ASS(200) + ABH (150) | 37,3* | 34,2*^ | 35,0*^ | 33,1*^ | 34,9*^ | 35,3*^ | 34,7*^ |
| IM(10) + AB(100) | 15,2* | 13,1*^ | 13,7*^ | 12,6*^ | 13,2*^ | 13,0*^ | 12,0*^ |
| NP(100) +QU (50) | 23,1* | 20,2*^ | 21,0*^ | 19,8*^ | 21,4*^ | 20,5*^ | 20,7*^ |
| VL(10) +SB(50) | 19,3* | 16,9*^ | 17,2*^ | 17,4*^ | 16,6*^ | 17,0*^ | 17,8*^ |
| (Anmerkung: AK = aktive Kohle, MTS = Magnesiumtrisilikat, MKZ = Mikrokristallzellylose, LAK = Laktose, FRU = Fruktose) | | | | | | | |

Die durchgeführte Untersuchung zeigt die Erhöhung der pharmakologischen Aktivität der Kombinationen der SEM mit Karbonsäuren, Aminosäuren, Vitaminen, Polyphenolverbindungen und den polyphenolenthaltenden Pflanzenauszügen unter der Wirkung der Kohlenhydrate und der Stoffe mit Sorptionsfähigkeiten.

**Beispiel 4:** Die klinische Untersuchung des Einflusses der Kohlenhydrate und der Stoffe mit Sorbtionsfähigkeiten auf die Auftretung der Nebenerscheinungen der SEM und ihrer Kombinationen mit Vitaminen, Karbonsäuren, Flavonoiden oder mit dem flavonoidenthaltenden Pflanzenauszug.

Die Untersuchungen wurden mit 14 ambulanten Patienten im Alter von 32 - 68 Jahren durchgeführt. Sie nahmen die SEM während des 4-5 Wochen andauernden Heilkurses anlässlich der Verschärfung der chronischen Entzündungskrankheiten ein. Zehn Patienten nahmen ASS in der Kombination mit Ammoniumsukzinat für die Reduzierung der Blutgerinnung nach einem erlebten Infarkt. Das Ammoniumsukzinat wurde in der Kombination für den Wiederaufbau des Energiewechsels der Miokarde benutzt. Ein Teil der Patienten (6 Menschen) nahmen Ortofen (0,05g) mit Vitaminen P (0,01g) und C (0,01g) anlässlich der Verschärfung der Rheumatoidarthritis (3 Mal pro Tag 5 Wochen lang) ein. Die Präparate wurden in Pulverform gegeben. Drei der untersuchten Patienten bekamen die Mischung von Präparaten mit Kartoffelstärke (1:1). Alle Patienten, die die Kombination mit der Stärke bekamen, vertrugen das Heilmittel besser. Sie hatten weniger ausgeprägte Dyspepsieerscheinungen, Kopfschmerzen und Schwindel. Eine Gruppe aus 8 Menschen bekam Indometacin mit dem flavonoidenenthaltenden Präparat "Carsil" (Legalon) anlässlich der Gicht (2 Menschen), anlässlich der Phlebitis (4 Menschen) oder anlässlich der Rheumatoidathritis (2 Menschen). Wie im vorigen Fall wurde das Präparat auch in Pulverform (0,05g Indometacin, 0,25g Legalon) 3 Mal pro Tag in der ersten Woche und 4 Mal pro Tag die letzten 4 Wochen eingenommen. Die Hälfte der Gruppe bekam das Präparat mit MKZ im Massenverhältnis 1:2. Alle Patienten, die mit dem kombinierten Präparat SEM und das Bioflavonoidsorbent MKZ bekamen, zeigten weniger ausgeprägte Indometacinnebenerscheinungen, wie z. B. Dyspepsieerscheinungen, Schwindel und Kopfschmerzen. Von der Gruppe, die kein Sorbent einnahm, musste ein Patient am Ende der dritten Woche wegen starker Epigastralgie und Erbrechens auf die Einnahme des Präparats verzichten. Ein weiterer Patient musste Antihistaminmittel einnehmen, um die Allergieerscheinungen zu reduzieren.

Die Hälfte der Patienten aus 10 Menschen bekam ASS als Antiaggregationsmittel zusammen mit Ammoniumsukzinat (0,25g und 0,1g entsprechend), und die zweite Hälfte bekam die genannte Kombination mit aktiver Kohle (0,35g) 2 Mal pro Tag 8 Wochen lang. Vier Menschen aus der Fünfgliedergruppe, die ein Sorbent einnahmen, zeigten die bessere Verträglichkeit der Mischung aus ASS und Ammoniumsukzinat (sie nahmen diese Kombination auch früher ein). Alle fünf Patienten zeigten weniger ausgeprägte Dyspepsieerscheinungen. Die Ergebnisse der Untersuchung beweisen daher die Reduzierung der Nebenerscheinungen bei gemeinsamer Anwendung des SEM mit den anderen biologisch aktiven Verbindungen und bei der Anwendung von Kohlehydraten oder Stoffen mit Sorptionsfähigkeiten.

**Beispiel 5:** Beispiele der rationellen Kombinationen der SEM mit Stoffen, die die pharmakologische Aktivität verstärken, und mit Sorbenten und Kohlehydraten.

Diese Kombinationen sind die Ergebnisse von versuchsweise durchgeführten Untersuchungen.

**Rezept 1:**

ASS 0,5g
Vitamin 0,1g
Glykose bis 0,5g
(diese Rezeptur wird leicht technologisch produziert).

**Rezept 2:**

ASS 0,5g
Rutin 0,1 g
Stärke 0,01g
Glykose bis 0,5g

(das Präparat wird leicht technologisch produziert und ist hoch wirksam)

**Rezept 3:**

Indometacin von 0,025 bis 0,05g
Silimarin 0,01g
Aktive Kohle von 0,02 bis 0,5g

(das Präparat besitzt hohe Wirksamkeit in allen Kompositionen und hat niedrige Toxizität) .

**Rezept 4:**

ASS 0,25g
Ammoniumsukzinat 0,1g
Auszug von Bergenium 0,1g
MKZ 0,05g

**Rezept 5:**

ASS 0,4g
Ammoniumsukzinat 0,1g
Auszug von Bergenium 0,03g
Askorbinsäure 0,03g

Glykose 0,04g

**Rezept 6:**

ASS 0,25g
Ammoniumsukzinat 0,15g
Süssholzauszug 0,05g
Askorbinsäure 0,05g
Glykose 0,1g

**Rezept 7:**

Ortofen 0,05g
Vitamin P 0,1g
Vitamin C 0,1g
Stärke 0,25g

**Rezept 8:**

Butadion 0,05g
Glutaminsäure 0,1g
Laktose 0,2g
Stärke 0,1g
Magnesiumstearat 0,05g

Industrielle Anwendbarkeit

Die pharmazeutischen Kompositionen gemäß der Erfindung können bei der Behandlung verschiedener Krankheiten, die die Anwendung der SEM fordern, benutzt werden. Diese pharmazeutischen Kompositionen aus verschiedenen Komponentenkombinationen, die die pharmakologische Wirkung der SEM verstärken und die Nebenerscheinungen reduzieren, geben die Möglichkeit, die Wirksamkeit der Behandlung der Kranken zu erhöhen und die Anzahl der negativen Wirkungen und Komplikationen der Patienten, die die SEM-Anwendungen fordern, herabzusetzen.

**Patentansprüche**

1. Pharmazeutische Komposition auf der Basis von steroidfreien entzündungswidrigen Mitteln, die ein steroidfreies entzündungshemmendes Mittel und eine seine pharmakologische Wirkung verstärkende und die Toxizität und die Nebenerscheinungen reduzierende Verbindung enthält,
   **dadurch gekennzeichnet,**
   **dass** die Komposition als Verbindung mindestens eine Verbindung aus folgender Gruppe doppelstämmiger Karbonsäuren oder ihre Salze, Aminosäuren oder ihre Salze, Polyphenolverbindungen oder polyphenolentaltende Produkte tierischer und / oder vegetarischer Herkunft, Stoffe mit Vitaminwirkung und zusätzlich mindestens einen Zielzusatzstoff (Kohlenhydrat und / oder Sorbent) enthält.

2. Pharmazeutische Komposition nach Anspruch 1,
   **dadurch gekennzeichnet,**
   **dass** sie als steroidfreie, entzündungshemmende Verbindung Azetylsalizylsäure enthält.

3. Pharmazeutische Komposition nach Anspruch 1 oder 2,
   **dadurch gekennzeichnet,**

**dass** sie als Produkt vegetarischer Herkunft mit Polyphenolverbindung den Auszug von Bergenium und / oder einen Süssholzauszug enthält.

4. Pharmazeutische Komposition nach einem der Ansprüche 1 bis 3,
   **dadurch gekennzeichnet,**
   **dass** sie als Stoff mit Vitaminwirkung Askorbinsäure enthält.

5. Pharmazeutische Komposition nach einem der Ansprüche 1 bis 4,
   **dadurch gekennzeichnet,**
   **dass** sie als Zielzusatzstoff Glykose enthält.

6. Pharmazeutische Komposition nach einem der Ansprüche 1 bis 5,
   **dadurch gekennzeichnet,**
   **dass** sie enthält (in Massenprozenten):

   - Azetylsalizylsäure        25-80
   - Ammoniumsukzinat        5-30
   - Auszug von Bergenium        5-20
   - Askorbinsäure        5-20
   - Glykose        Rest

7. Pharmazeutische Komposition nach einem der Ansprüche 1 bis 6,
   **dadurch gekennzeichnet,**
   **dass** sie als Karbonsäure Bernsteinsäure enthält.

8. Pharmazeutische Komposition nach einem der Ansprüche 1 bis 7,
   **dadurch gekennzeichnet,**
   **dass** sie zusätzlich einen pharmakologisch passenden Träger, der für die Oraleinführung geeignet ist, enthält.

9. Pharmazeutische Komposition nach einem der Ansprüche 1 bis 8,
   **dadurch gekennzeichnet,**
   **dass** sie die Form einer Tablette oder Kapsel hat.

## INTERNATIONAL SEARCH REPORT

| International application No. |
| --- |
| RUO.1/00132 |

**A. CLASSIFICATION OF SUBJECT MATTER**

A61K31/616, 31/194, 31/375, 31/7004, 35/78, A61P 29/00

According to International Patent Classification (IPC) or to both national classification and IPC

**B. FIELDS SEARCHED**

Minimum documentation searched (classification system followed by classification symbols)

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>A | RU 2128997 C1 (KAZANSKOE PROIZVODSTENNOE KHIMIKO FARMATSEVTICHESKOE OBIEDINENIE "TATKHIMFARPRE_PARATY")<br><br>the abstract, the claims, the description, column 3 | 1,2,4,5,7_9<br>3,6 |
| X<br>A | RU 94041221 A1 (PFEIZER; INC:) 1996.07.10<br>the abstract, the description, pages 4,5. | 1,2,4,57,9<br>3,6 |
| X<br>A | WO 8904165 A (HAKLITCH JOSEPH A ET AL.)<br>1989-05-18 | 1,2,3,4,6-9 |

☐ Further documents are listed in the continuation of Box C.    ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 15 June 2001 (15.06.2001) | 21 June 2001 (21.06.2001) |

| Name and mailing address of the ISA/ | Authorized officer |
| --- | --- |
| Facsimile No. | Telephone No. |

Form PCT/ISA/210 (second sheet) (July 1992)